# EUROPEAN PATENT APPLICATION

(11) **EP 3 574 886 A1**
(43) Date of publication of application: **04.12.2019**
(21) Application number: 18382381.4
(22) Date of filing: 01.06.2018
(51) Int. Cl.: A61J 1/05, A61C 19/00

(54) **MEDICAL CONTAINER**

(71) Applicant: Tabuenca Huerta, Joaquin, 28229 Villanueva del Pardillo (ES)
(72) Inventor: TABUENCA HUERTA, Joaquín, 28229 Villanueava del Pardillo (ES); SANTIAGO, Daniel Palma, Veranda/Siete Soles, Malagueño Provincia de Córdoba (AR)
(74) Representative: Carlos Hernando, Borja

(57) **Abstract**

The invention relates to a medical container for the application of medical repair material for the subsequent placement thereof in a patient by using a scoop or spoon.

## Description

### Technical field

This invention relates to a medical container from which a medical repair material of high biological and economic value, mixed or not with other components, will be applied in small quantities in a patient by using a scoop or spoon handled manually by a health professional. The container object of the invention allows the medical professional to collect the material in small quantities by handling the spoon or scoop with one hand, without any external help, so that he can use the other hand for other actions. Thanks to the structure of the container it prevents the material contained therein from falling out when it is extracted, thus facilitating the collection and extraction thereof with the scoop or spoon.

The container is preferably used for applying different materials, such as autologous or self-grafting materials (from the patient himself), xenografts (purchased and not from the patient), bone regenerators, blood plasma, patient's own bone, etc. This material can be a single material or a mixture thereof and is applied directly to the required area of the patient, by the use of the mentioned spoon or scoop.

The invention is preferably applied in the dental and dentistry field, although it can also be used in other medical specialties such as traumatology.

### Background of the invention

In the state of the art, different instruments of different sizes and shapes for collecting various repair materials are known, and in particular, various containers of circular base are known, usually in the dental sector, from which a material is collected for subsequent application thereof in a patient's teeth. In this container different materials can be mixed, or they can be inserted directly into the same after mixing it and before its application in the patient. This application is carried out manually with the help of a small scoop or spoon that is inserted into the patient's mouth. The material or materials inserted in these containers are scarce materials so they have a great biological and economic value.

Once the mixture is made in the container, or transferred to the same, the health professional inserts the scoop or spoon in the container to extract the material and apply it in the teeth. This manoeuvre requires, due to the configuration of the current containers, that the health professional (surgeons, nurses, etc.) use both hands, and even have the help of a third person, to take the material from the bottom of the container and remove it from the same. For example, the healthcare professional can grab a spoon with one hand, a tool with the other to push the material over the spoon and they would need a third hand to hold the container. Alternatively, the container will be attached to a surface and the handling thereof will not be so simple.

Also, the professional can hold the container with one hand and the other will hold the spoon, in order to get the specific amount of material, which normally does not exceed 2 grams, although it may vary depending on the size of the defect to be repaired, as well as the medical specialty in which it is being used, because when using the spoon to get the material from the bottom of the container, if the container does not tilt with the other hand, the material can fall, which may sometimes lead to the loss of material, or materials, which is sometimes irreplaceable given the biological origin and scarcity thereof, without despising the high economic value of the material.

The above is due to the fact that the upper ends of the container walls are perpendicular to the support surface, so that by sliding the spoon through the end of said wall, dragging the material contained in the spoon, it can fall out of the container when reaching said upper end, with the consequent loss of material. This material dragged from the bottom of the container to the upper end of the walls always tends to pour out, and therefore this problem is solved at present by using two hands, one to hold and tilt the container and another to handle the spoon or by another instrument handled by the same health professional or other health personnel. In other words, a first instrument is used to collect the material of the container and to drag it from the bottom to the upper end and with a second instrument it is pushed, once it has reached the upper end, to the material so that it does not fall out of the container and in turn is available on the first instrument. This solution avoids the spillage of the product, but it makes the operation difficult and expensive by requiring two hands of qualified personnel.

Therefore, the present invention aims to simplify the operation of applying repair material in a denture or bone, saving human time and material and thus reducing the economic and material expenses in certain health procedures, by developing a new container for the application of a repair material.

### Description of the invention

The present invention proposes, according to a first object of the invention, a medical container for the application of repairing material in the dental field or other field of the medicine, according to the claim 1, in particular a container with a base, an inner wall and at least one perimeter wall that has at the upper end thereof a higher section that determines the mouth of the container, opposite the base, the inner wall of the container being inclined so that the upper section converges towards the Inside the container, thereby determining an area in the mouth of the container, that is, at the free end of the upper section, which is less than the area determined by the inner wall at the beginning of the upper section wherein the inner wall of the container is inclined.

Therefore, the container is formed by a base and a lateral perimeter wall, if the base is a curved body, or formed by several lateral perimeter walls, if the base is a body with more than three sides, said wall emerging in both cases from the base, and leaving the upper end thereof, or mouth, open. The upper section of the wall equivalent to the upper end of the wall that determines the mouth of the container, or walls, preferably has along the entire perimeter thereof an angle converging towards the inside of the container, this angle having a value of less than 90°, preferably between 45° and 85°, measured by the inside of the container between the horizontal base thereof and a perpendicular with said horizontal base. Obviously, if measured from the outside of the container, the angle must have complementary dimensions with the previous one, i.e. greater than 90°, preferably between 95° and 135°.

This angle of the upper end of the wall, or walls, of the container, enables, by the use of a scoop or spoon to collect the repair material contained inside the container along with the action of the gravitational force, the material that is dragged by the spoon to move towards the centre pushed by the converging end of the wall or walls, so that it is possible to collect the desired material from the inside of the aforementioned container without the need of external aid. This difference between the present invention and the known containers for the application of repair material, preferably dental, enables the problems described above to be overcome definitively.

Additional and complementary features are cited in dependent claims, as for example:
- Outer surface of the wall of the concave container wall to facilitate holding thereof with the fingers of the hand,
- Inner surface of the wall inclined with respect to the bottom of the base of the container to push the repair material from the bottom with a spoon and to facilitate the drag thereof to the mouth of the container,
- Inner surface of the wall perpendicular to the bottom of the base of the container,
- Base preferably circular, although it could also be oval, triangular, rectangular, etc.
- Notches or inlets in the mouth of the container, preferably two in diametrically opposite points, in the case of circular mouth, for the support of the handle of the scoop, spoon or instrument used for collecting and applying the repair material,
- The material of the container should preferably allow the sterilization thereof so that different plastic or metal materials can be used, although it is also possible to use more economical materials, paper or cardboard, when the container is a single use, exclusively for use in a single surgical act, and
- The base of the container will preferably contain more material in order to increase the weight thereof and prevent it from moving, or it could even be magnetized, so as to avoid risks of spillage of repair material due to unwanted and uncontrolled movement of the container during use thereof, in addition to preventing the container from moving and allowing it to be used with a single hand by the medical professional. It will be heavier than the rest.

### Brief description of the drawings

The above and other advantages and characteristics of the invention will be more fully understood from the following detailed description of the embodiments, with reference to the attached figures, which should be considered in an illustrative and non-limiting manner, wherein:
Figure 1 shows a perspective view of an exemplary embodiment of a container for the application of dental material.
Figure 2 shows a top view of the container of Figure 1.
Figure 3 shows a cross-section according to the CC cut of the container of Figure 2.
Figure 4 shows three stages during the use of the container object of the present invention. Stage a) shows the final location of a scoop that has removed the repair material of the container; stage b) shows the scoop by dragging the material from the bottom of the container and through the inclined inner wall thereof; and stage c) shows the material on the scoop after resting on the upper section of the mouth of the container, prior to the final location.

### Detailed description of preferred embodiments

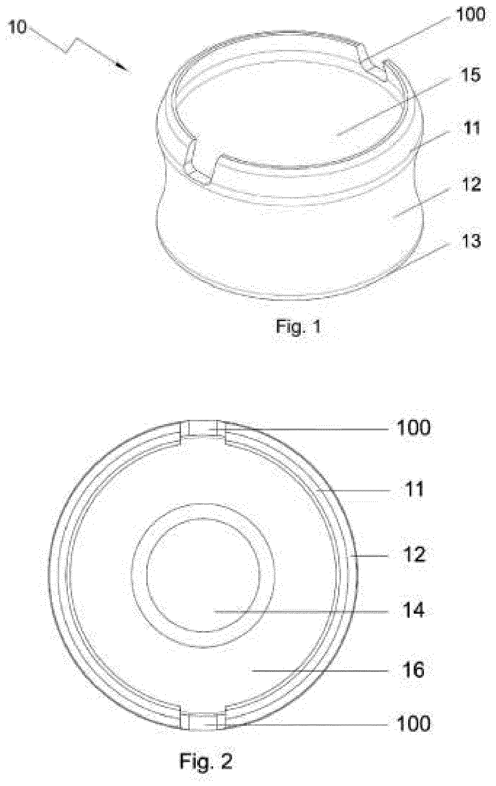

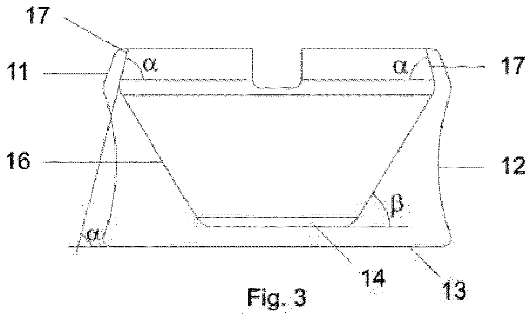

According to Figures 1 to 4, an exemplary embodiment of the container for the application of repair material, with a scoop or spoon for medical use, will be described below.

The container 10 preferably has a substantially cylindrical body, with a circular base 13, for the support on a surface and at least one perimeter wall 12, with an inner surface 16, wherein the upper end of the wall that determines the mouth 15 of the container 10 has an upper section 11 whose inner surface 16 is inclined an angle α, measured by the inside of the container, which converges towards the inside of the container 10, said inclination of the upper section 17 preventing the material from falling from the spoon or scoop when removing it from container. The mouth of the container 10 must have a sufficiently wide area to enable the insertion and handling of the scoop or spoon into the container 10. Likewise, the area of the mouth 15 of the container 10, determined by the inner surface 16 at the free end or upper end of the upper section 17 of the container 10, is less than the area determined by the inner surface 16 of the perimeter wall in the place wherein the upper section 17 starts, in other words, the place wherein the inclination of the inner surface 16 of the container 10 towards the inside of the container changes.

Said angle α is less than 90° measured with respect to the base of the container, preferably located between 45° and 85°, and more preferably approximately 70°. In the figures the location of this angle α is observed, in particular, between the surface wherein the container is supported and the perpendicular to said support surface. If said angle is measured by the inside of the container, it will be less than 90°, and if measured by the outside of the container, it will be greater than 90°.

Likewise, the outer surface of the wall 12 is preferably concave to facilitate the holding of the container with two fingers, thereby adapting to the curved shape of the fingers.

The inner surface 16 of the wall 12 is inclined with respect to the bottom 14 of the container, enabling this inner inclination to facilitate the drag of the repairing material along the inside 16 of the container 10 with the help of a spoon or scoop until it reaches the upper section 11, wherein by changing the inclination of the inside of the container 10, it is possible that the repair material remains on the scoop or spoon before removing it from the container 10.

The bottom 14 preferably has an area that is smaller than the opening area 15, the inclined inner surface 16 of the wall 12 of the container 10 thereby forming an angle with the bottom 14, measured by the outside of the container, preferably an angle β of between 0° and 90°, preferably between 15° and 70°, and more preferably of approximately 55°, to thus make it easier for the spoon or scoop to collect the material of the bottom 14 of the container which is then dragged along the inner surface 16 of the wall 12.

The container 10 can preferably also include two grooves or recesses 100 located in the upper section 17, and preferably opposite on the section, which enable the scoop or spoon to rest on the container 10 when it is not in use. When the container 10 is substantially cylindrical, the grooves will preferably be diametrically opposite.

As is evident, the wall 12 of the container depends on the shape of the base 13, so that if the base 13 is circular, as is preferred, the container 10 will only comprise a perimeter wall. However, the base 13 can also have at least 3 sides, such that the container will have three walls 12, while if, for example, the base 13 has four or five sides, the container will have four or five walls, respectively.

The container is preferably made of a material that can be sterilized, preferably metal, ceramic, vitreous or plastic. In any case, other materials can be used if the container 10 is designed for a single use, it being able to be made of paper or cardboard. Likewise, the base 13 of the container is overweight to prevent or hinder the container 10 from moving during handling. Alternatively, the base can incorporate a magnetic material so that it remains attached to a surface. In addition, the container 10 may include in the base 13 thereof attachment means which are complemented by other means arranged on the support surface thereof, such as an inner or outer thread.

The dimensions of the container will be variable, but preferably, a container 10 for repair material in the dental sector will comprise a circular base, giving rise to a substantially cylindrical body, with a height of approximately 20 mm, a base diameter of approximately 35 mm, a top diameter of the mouth of approximately 30mm, a bottom diameter of approximately 15mm, a diameter of approximately 32 mm in the place wherein the inclination of the inner surface 16 of the wall 12 changes to the inclination of the upper section 11, and a separation of approximately 2 mm between the bottom 16 and the base 13.

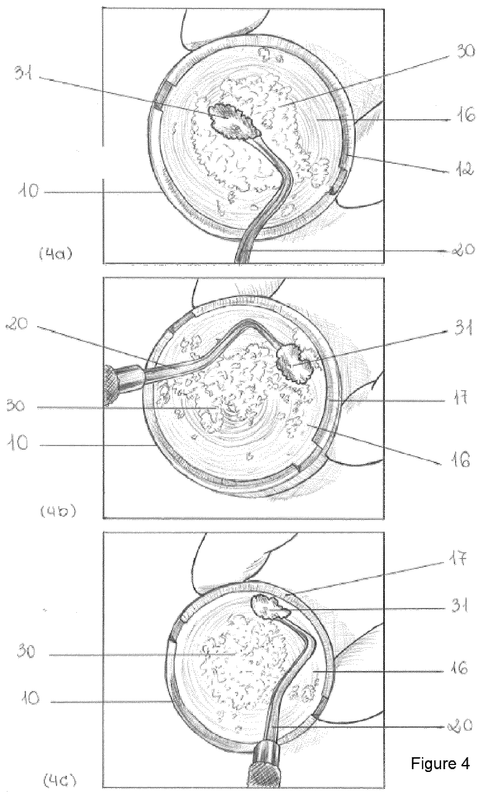

Figure 4 shows three top images of the container 10 object of the invention in three different stages during use. In Figure a), the scoop 20 is observed with a small amount of repair material 31 at its end after having collected the amount of the material 30 inserted into the container 10. In Figure b), at a stage prior to stage a, the scoop 20 drags the amount of previously collected material 31 along the inclined surface 16 of the wall 12 with the help of scoop 20. In the third and last image c), it is observed, in the stage after stage b), and before stage a), how the scoop 20 with the material 31 rests on the inclined section 17 of the container 10, achieving that this material 31 remains on the scoop 20 without risk of spilling when the scoop 20 ceases to be in contact with the container 10.

## Claims

1. A medical container (10) for the application of medical repair material (30) comprising a base (13), an inner surface (16) and at least one perimeter wall (12), **characterised in that** the upper end, opposite the base, of the inner surface of the wall (12) which determines the mouth (15) of the container (10) has an upper section (17) wherein the inner surface (16) of said section (17) is inclined an angle (α) that converges towards the inside of the container (10), determining an area in the mouth (15) of the container (10) smaller than the area determined by the inner wall (16) at the beginning of the upper section wherein the inclination of the inner wall (16) of the container (10) occurs.

2. The container, according to claim 1, **characterised in that** the angle (α), measured with respect to the base of the container, is less than 90°.

3. The container, according to claim 2, **characterised in that** the angle is between 45° and 85°.

4. The container, according to any of the claims, **characterised in that** the outer surface of the wall has a concave surface.

5. The container, according to any of the claims, **characterised in that** it comprises a bottom whose area is smaller than the area of the mouth.

6. The container, according to claim 5, **characterised in that** the inner surface of the wall forms an acute angle with the bottom measured by the outside of the container.

7. The container, according to any of the preceding claims, **characterised in that** it comprises two grooves in the upper section.

8. The container, according to any of the claims, **characterised in that** the base is circular, determined a single wall.

9. The container, according to any of the claims, **characterised in that** the base comprises at least three sides determining at least three walls, one on each side.

10. The container, according to any of the preceding claims, **characterised in that** it is of a sterilisable material.

11. The container, according to any of the preceding claims, **characterised in that** the base comprises an additional weight.
